(19) **Europäisches Patentamt / European Patent Office / Office européen des brevets**

(11) **EP 4 624 044 A1**

(12) **EUROPEAN PATENT APPLICATION**
published in accordance with Art. 153(4) EPC

(43) Date of publication:
01.10.2025 Bulletin 2025/40

(21) Application number: 23953916.6

(22) Date of filing: 31.10.2023

(51) International Patent Classification (IPC):
*B01J 29/44* (2006.01)  *C10G 1/10* (2006.01)
*C10G 2/00* (2006.01)  *B01J 29/76* (2006.01)
*B01J 29/48* (2006.01)  *B01J 29/40* (2006.01)
*B01J 29/46* (2006.01)  *C07C 2/86* (2006.01)
*C07C 15/06* (2006.01)  *C07C 15/04* (2006.01)
*C07C 15/08* (2006.01)

(86) International application number:
PCT/CN2023/128160

(87) International publication number:
WO 2025/065781 (03.04.2025 Gazette 2025/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 26.09.2023 CN 202311252638

(71) Applicant: Dalian Institute of Chemical Physics,
Chinese Academy of Sciences
Dalian, Liaoning 116023 (CN)

(72) Inventors:
• PAN, Xiulian
Dalian, Liaoning 116023 (CN)
• DING, Yi
Dalian, Liaoning 116023 (CN)
• BAO, Xinhe
Dalian, Liaoning 116023 (CN)

(74) Representative: Murgitroyd & Company
165-169 Scotland Street
Glasgow G5 8PL (GB)

(54) **METHOD FOR PREPARING AROMATIC HYDROCARBONS BY MEANS OF COUPLING CONVERSION OF CO AND POLYOLEFIN**

(57) Provided is a method for preparing aromatic hydrocarbons by means of coupling conversion of carbon monoxide and a polyolefin, belonging to the technical field of polyolefin plastic conversion and recycling. The method uses carbon monoxide and polyolefin type plastic as reaction raw materials, uses as a catalyst a bifunctional catalyst consisting of metal, a metal oxide and a molecular sieve, and comprises performing a conversion reaction under the action of the catalyst. Because carbon monoxide may come from carbon resources such as coal, biomass and natural gas, the reaction process not only achieves optimized utilization of carbon resources, but also converts polyolefin waste plastic into valuable resources, achieving high-value utilization and producing aromatic hydrocarbon products having high added value. The reaction has a very high product yield and product selectivity, the yield of the aromatic hydrocarbons can reach 50%-80%, the proportion of benzene, toluene and xylene in the aromatic hydrocarbons is greater than 60%, and the catalyst can be repeatedly used after simple regeneration treatment. The present application is a new technology for comprehensive utilization of polyolefin type waste plastic and carbon monoxide utilization, and has a good application prospect.

EP 4 624 044 A1

**Description**

**TECHNICAL FIELD**

The present disclosure belongs to the technical field of high-value aromatic chemicals preparation via coupling conversion of CO and polyolefin, in particular to a catalyst and a method for preparing aromatic hydrocarbon by coupling conversion of CO and polyolefin.

**BACKGROUND**

Plastics are essential organic synthetic polymer materials widely used in packaging, agriculture, construction, automotive, and other fields. By 2019, global plastic production had reached 460 million tons per year, making it one of the most produced synthetic materials. Among these, polyolefin plastics, such as polyethylene and polypropylene, account for approximately 55% of total plastic production. However, due to the chemical inertness of polyolefin, a significant portion of polyolefin-based single-use products are difficult to recycle, leading to severe environmental pollution that has attracted widespread attention from researchers.

For polyolefin, the $C(sp^3)$-$C(sp^3)$ bonds in their backbone are highly stable and resistant to cleavage. Therefore, recycling polyolefin requires additional energy to cleave these C-C bonds. However, traditional polyolefin recycling methods, such as pyrolysis and hydrogenolysis, result in random and non-selective C-C bond cleavage, producing pyrolysis oils dominated by long-chain alkanes or olefins with a wide boiling range and relatively low added value. These methods cannot produce high-value-added products with high selectivity, making plastic pyrolysis economically unviable due to the lack of a feasible driving force.

Carbon monoxide (CO) molecules are unsaturated and metastable, and are chemically stable in terms of decomposition. At room temperature, CO does not react with acids or bases but can form explosive mixtures when mixed with air. It is highly flammable and explosive upon exposure to open flames or high temperatures. The carbon atom in CO molecules has an oxidation state of +2, allowing it to be oxidized to an oxidation state of +4 (exhibiting reducing properties) or to be reduced to lower oxidation states (exhibiting oxidizing properties). As a major component of syngas and various types of coal gas, CO is a crucial raw material for synthesizing a wide range of basic organic chemical products and intermediates. Starting from CO, almost all basic chemicals can be produced, including ammonia, phosgene, alcohols, acids, anhydrides, esters, aldehydes, ethers, amines, alkanes, and alkenes. Additionally, CO can react with transition metals to form metal carbonyls or derivatives thereof, which can be used as a homogeneous catalyst in organic chemical production. Moreover, CO can also be employed as a termination agent in polyethylene polymerization reactions. Although some studies have shown that high-value chemicals or liquid fuels (e.g., light olefins, aromatics, or higher alcohols) can be produced from CO and $H_2$ under high temperature and pressure, current $H_2$ production heavily relies on fossil fuels (coal, petroleum, and natural gas). Without substantial "green hydrogen" production technologies, extensive use of $H_2$ resources implies increased $CO_2$ emissions, which is detrimental to achieving carbon peak and carbon neutrality goals. Therefore, utilizing CO without $H_2$ is more attractive and more challenging.

Aromatics, particularly benzene, toluene, and xylene (BTX), are high-demand basic organic chemical raw materials, typically obtained from naphtha through catalytic reforming and distillation. Using waste polyolefin as raw materials to produce aromatics via catalytic pyrolysis under mild conditions is an economically viable technical route. Researchers such as Zhang et al. from the University of California, Santa Barbara, used a Pt/$\gamma$-$Al_2O_3$ catalyst to convert polyolefin into long-chain alkylaromatics at 280 °C, achieving an aromatic yield exceeding 70%. Although this method enables conversion at a relatively mild temperature (280 °C), the products contain almost no higher-value benzene, toluene, or xylene (Fan Zhang et al., Science 370 (2020) 437-441). There have been no reports on the coupling conversion reaction of CO and polyolefin to produce high-value aromatics at lower temperatures, such as below 300 °C.

**SUMMARY OF THE INVENTION**

In view of the aforementioned problems, the present disclosure provides a catalyst and a method for preparing aromatic hydrocarbon by coupling conversion of CO and polyolefin.

**The technical** solutions of the disclosure are as follows:

The present disclosure provides a catalyst for preparing aromatic hydrocarbon by coupling conversion of CO and polyolefin. The catalyst is a bifunctional catalyst including a metal and a zeolite. Taking CO and polyolefin as reaction raw materials, the conversion reaction is carried out in a tank reactor under the action of the catalyst. One functional component of the bifunctional catalyst is selected from a metal, a metal oxide, and a mixture thereof. Another functional component includes a zeolite. The functional component of metal/metal oxide activates $CO_2$, while the zeolite activates the polyolefin. The zeolite has an MFI, MEL, or MWW topological structure.

Based on the above technical solutions, the metal/metal oxide preferably includes one or more elements selected from Fe, Co, Ni, Mn, Ce, Cu, Ru, Zn, Ga, Rh, Pd, Ir, and Pt. A mass fraction of the metal/metal oxide in the bifunctional catalyst is 0.1-60 wt%, preferably 0.5-50 wt%.

The metal/metal oxide is either directly loaded onto the zeolite or is dispersed on a metal oxide carrier and then physically mixed with the zeolite to form the bifunctional catalyst. The metal oxide carrier is selecte4d from $Al_2O_3$, $TiO_2$, $ZrO_2$, $CeO_2$, ZnO, $MoO_x$, and $MnO_x$, and mixtures thereof.

Based on the above technical solutions, the zeolite having an MFI, MEL, or MWW topological structure preferably has a framework including at least one of Si-O, Si-O-Al, Si-O-Al-P, Si-O-Al-B, and Si-O-Al-Ge. The zeolite having an MFI, MEL, or MWW topological structure is preferably at least one of MCM-22, ZSM-11 and ZSM-5.

Based on the above technical solutions, the zeolite having an MFI, MEL, or MWW topological structure preferably contains moderate-strength acid sites in an amount of $\geq 0.1$ mol/kg, more preferably $\geq 0.2$ mol/kg, and further preferably $\geq 0.25$ mol/kg.

The acid strength is defined by $NH_3$-TPD peaks, including three types of weak acid, moderate-strength acid, and strong acid. $NH_3$-TPD is based on desorption peak positions of $NH_3$ under standard test conditions. The standard test conditions are that a ratio (w/f) of sample mass w and carrier gas flow rate f is 100 g·h/L and a heating rate is 10 °C/min, a TCD records a thermal conductivity signal of desorption of $NH_3$ and draws a desorption curve. The inorganic solid is classified into three acid strength categories based on the peak positions. Weak acid refers to acid sites with $NH_3$ deposition temperatures below 275 °C; moderate-strength acid refers to acid sites with $NH_3$ deposition temperatures between 275 °C and 500 °C; and strong acid refers to acid sites with $NH_3$ deposition temperatures above 500 °C. The zeolite may be either laboratory-synthesized or commercially purchased, provided it meets the requirements of the present disclosure.

Based on the above technical solutions, preferably, the metal or metal oxide is compounded with the zeolite in a mechanical mixing or loading mode. The metal/metal oxide is preferably compounded with the zeolite via impregnation, deposition-precipitation, vapor deposition, or physical mixing.

Based on the above technical solutions, the bifunctional catalyst is preferably pre-reduced in $H_2$ or CO atmosphere at 300-500 °C for 0.5-10 h.

Based on the above technical solutions, the present disclosure further provides a method for preparing aromatic hydrocarbon by coupling conversion of CO and polyolefin. The method uses CO and polyolefin as reaction raw materials to carry out the conversion reaction in a tank reactor employing the bifunctional catalyst provided in the aforementioned technical solutions.

Based on the above technical solutions, the polyolefin includes at least one of polyethylene, polypropylene, and polybutene, as well as plastic products made therefrom or post-consumer waste plastics.

Based on the above technical solutions, a pressure of the CO is preferably 0.2-6.0 MPa, more preferably 0.5-3.0 MPa; a reaction temperature is 180-400 °C, preferably 230-300 °C; a mass ratio mass ratio of catalyst to polyolefin is $\geq$ 1:500, preferably $\geq$ 1:100.

Based on the above technical solutions, when the reaction is carried out in a tank reactor, a residence time of reactants is 0.5-20 h, preferably 1-10h, more preferably 2-10h.

Based on the above technical solutions, when the reaction is carried out in a moving-bed reactor, a gas hourly space velocity is 20-2000 mL·g$^{-1}$·h$^{-1}$, preferably 20-1000 mL·g$^{-1}$·h$^{-1}$, more preferably 80-1000 mL·g$^{-1}$·h$^{-1}$.

The present disclosure has the following advantages:

1. Different from traditional plastic pyrolysis technologies, the present disclosure employs a composite catalyst to achieve one-step coupling of CO and polyolefin for highly selective conversion into high-value aromatic such as benzene, toluene, and xylene. The introduction of CO not only serves as a raw material for aromatic production but also reacts with hydrogen species generated during the aromatization of hydrogen transfer, suppressing the alkane formation and enhancing the selectivity of aromatic components.

2. Different from mechanisms of high-temperature polyolefin pyrolysis, at low temperatures (reaction temperature below 300 °C), polyolefin cannot generate aromatics through dehydrogenation aromatization (Equation 1) but can only through aromatization of hydrogen transfer (Equation 2). Therefore, the yield of aromatics at low temperatures is limited by this reaction mechanism and cannot exceed 50%.

$$C_nH_{2n} \rightarrow \frac{n}{6} C_6H_6 + \frac{n}{2} H_2 \tag{1}$$

$$C_nH_{2n} \rightarrow \frac{n}{3x+6} C_6H_6 + \frac{3n}{3x+6} C_xH_{2x+2} \quad (x \geq 2) \tag{2}$$

The present disclosure utilizes hydrogen spillover between catalyst components and utilizes CO to consume hydrogen spilled over from zeolite hydrogen transfer aromatization, preventing its conversion into alkanes. This breaks the original reaction equilibrium, and enables highly selective production of value-added aromatic products under relatively mild reaction conditions (reaction temperature below 300 °C), demonstrating strong economic application prospects.

3. The present disclosure uses polyolefin as raw materials, which may also include plastic products made from polyolefin and polyolefin-based materials, such as plastic bags, plastic barrels, plastic wrap, various films, waste food packaging, etc. The raw materials for the method of the present disclosure are widely available and exhibit high utilization rate, making it an effective and scalable polyolefin utilization solution.

4. The present disclosure cannot only applied to the resource utilization of CO emissions from processes such as oil refining, coking and ironmaking in the metallurgical industry, but also to the resource utilization of high-concentration CO-containing emissions from processes such as ammonia synthesis and methanol production in the chemical industry. It can even be applied to the resource utilization of emissions containing both CO and $CO_2$. With broad raw material sources and strong applicability, the present disclosure significantly promotes the utilization of CO while reducing $CO_2$ emissions.

5. After regeneration by means of calcination in air followed by hydrogen reduction, the catalyst of the present disclosure can be recycled, with no significant catalyst loss, significantly reducing costs associated with catalyst deactivation.

6. The preparation process of the nano-composite catalyst in the present disclosure is simple and operates under mild conditions. Moreover, the reaction process not only achieves the high-value utilization of both polyolefin and CO but also exhibits high space-time yield and selectivity. The yield of mixed aromatics may reach 50-80%, with benzene, toluene, and xylene accounting for over 60% of the aromatic products. Compared to traditional polyolefin pyrolysis under inert gas conditions, the aromatic yield is more than twice.

## DETAILED DESCRIPTION OF PREFERRED EMBODIMENTS

The following examples are provided to further illustrate the present disclosure, but the scope of the claims is not limited by the examples. Meanwhile, the examples only give some conditions for achieving the objectives, which does not mean that the conditions must be satisfied to achieve the objectives.

1. Preparation of zeolites

I . Preparation of zeolites

The moderate-strength acid sites described in the present disclosure can be characterized by various methods including but not limited to solid-state NMR ($^1$H NMR), $NH_3$-TPD (Temperature-Programmed Desorption), infrared spectroscopy (IR), and chemical titration.

The zeolite with an MFI, MEL, or MWW topology structure described in the present disclosure can be prepared by various methods and conditions. This patent exemplifies their preparation through hydrothermal synthesis.

1) The preparation process of a zeolite with an MFI topology structure can refer to the following method:

The raw materials, including 30% (mass concentration) silica sol, aluminum sulfate, sodium hydroxide, tetrapropylammonium hydroxide (R) and deionized water, were weighed at a oxide mass ratio of $SiO_2$:$Al_2O_3$:$Na_2O$:R:$H_2O$=5:0.02:2:1.5:200, and were stirred and aged at 30 °C for 2 h to obtain a product, then the product was transferred to a hydrothermal reactor for crystallization at 180 °C for 48 hours. The crystallized product was quenched to room temperature in a water bath, and then was repeatedly centrifuged and washed until the supernatant reached pH 7. The precipitate was dried at 110 °C for 17 hours and then was calcined in air at 600 °C for 3 hours to obtain a ZSM-5 molecular sieve with topology structure, labeled as zeolite 1.

2) The preparation process of a zeolite with an MFL topology structure can refer to the following method:

The raw materials, including 30% (mass concentration) silica sol, aluminum isopropylate, sodium hydroxide, tetrabutylammonium hydroxide (R) and deionized water were weighed at a mass ratio of $SiO_2$:$Al_2O_3$:$Na_2O$:R:$H_2O$=10:0.3:1:2:200, and were stirred overnight at room temperature to obtain a product, and then the product was placed in an oven at 65 °C for drying. The dried product was crushed and placed in a hydrothermal reactor. A certain amount of deionized water was poured in the reactor for crystallization at 170°C for 3 days. The crystallized product was filtered under suction, washed, dried, and subjected to ammonium exchange, then was calcined in a muffle furnace at 600 °C for 6 hours to obtain a ZSM-11 molecular sieve with an MFL topology structure, labeled as zeolite 2.

The zeolite having an MEL, MFI or MWW topological structure has a framework including at least one of Si-O, Si-Al-O, Si-Al-P-O, Si-Al-B-O, Si-Al-Ge-O.

Table 1 Preparation and performance parameters of zeolites with an MFI, MEL or MWW topological structure

| Sample No. | Type of zeolite | Source | Topology Structure | Amount of moderate-strength acid (mol/kg) | Pore volume, $V_p$ (cm$^3$/g) |
|---|---|---|---|---|---|
| Zeolite 1 | ZSM-5 | Hydrothermal synthesis | MFI | 0.87 | 0.35 |
| Zeolite 2 | ZSM-11 | Hydrothermal synthesis | MEL | 0.65 | 0.38 |
| Zeolite 3 | ZSM-5 | Purchased from Nankai University | MFI | 0.68 | 0.31 |
| Zeolite 4 | MCM-22 | Purchased from Nankai University | MWW | 0.43 | 0.36 |
| Zeolite 5 | SAPO-34 | Purchased from Nankai University | CHA | 0.36 | 0.32 |
| Zeolite 6 | USY | Purchased from Nankai University | FAU | 0.96 | 0.41 |
| Zeolite 7 | MOR | Purchased from Nankai University | MOR | 0.73 | 0.35 |
| Zeolite 8 | SAPO-11 | Purchased from Nankai University | AEL | 0.25 | 0.32 |
| Zeolite 9 | ZSM-5 | Purchased from Nankai University | MFI | 0.07 | 0.29 |

II. Preparation of bifunctional catalysts

The component of metals/metal oxide is combined with the zeolite through methods such as impregnation, deposition-precipitation, vapor deposition, or physical mixing to form bifunctional catalysts. Here, the bifunctional catalyst prepared via impregnation is taken as an example.

The impregnation method can be carried out using either incipient wetness impregnation or excess solution impregnation, as described below:

Dissolve the metal source in deionized water to prepare a precursor solution. The zeolite is impregnated in the precursor solution and thoroughly stirred to ensure uniform dispersion of the precursor solution and its solutes on the zeolite, followed by drying and calcination to obtain the desired metal oxide-modified zeolite.

A pore volume $V_p$ of the zeolite needs to be measured in advance before impregnation. If the incipient wetness impregnation is used, the volume of the precursor solution used $V_0$ should be equal to $V_p$. If the excessive impregnation method is used, the volume of the precursor solution used $V_0$ should be less than $V_p$.

The catalyst preparation parameters are shown in Table 2.

Table 2 Catalysts prepared by impregnation method and parameter characteristics thereof

| Catalyst No. | Metal | Zeolite component | Mass Fraction of metal component (wt%) | Impregnation, drying and calcination methods | | | |
|---|---|---|---|---|---|---|---|
| | | | | Impregnation method | Drying Temperature (°C), Time (h) | Calcined Temperature (°C), Time (h) | Reduction Temperature (°C), Time (h) |
| Catalyst A | Pt | Zeolite 1 | 1 | Incipient wetness impregnation | 120, 12 | 550, 6 | 330, 2 |
| Catalyst B | Pt + Zn | Zeolite 2 | 0.5 + 3 | Excessive Solution Impregnation | 80, 12 | 600, 4 | 400, 2 |
| Catalyst C | Pd | Zeolite 3 | 2 | Excessive Solution Impregnation | 70, 24 | 550, 6 | 300, 1 |
| Catalyst D | Fe | Zeolite 4 | 6 | Excessive Solution Impregnation | 100, 8 | 550, 6 | 350, 2 |
| Catalyst E | Mn | Zeolite 3 | 8 | Incipient wetness impregnation | 60, 24 | 600, 6 | 450, 1 |

(continued)

| Catalyst No. | Metal | Zeolite component | Mass Fraction of metal component (wt%) | Impregnation, drying and calcination methods | | | |
|---|---|---|---|---|---|---|---|
| | | | | Impregnation method | Drying Temperature (°C), Time (h) | Calcined Temperature (°C), Time (h) | Reduction Temperature (°C), Time (h) |
| Catalyst F | Ga | Zeolite 1 | 5 | Excessive Solution Impregnation | 120, 10 | 550, 6 | 400, 1 |
| Catalyst G | Ni | Zeolite 4 | 6 | Excessive Solution Impregnation | 90, 8 | 550, 4 | 300, 4 |
| Catalyst H | Ir + Mn | Zeolite 2 | 0.5 + 5 | Incipient wetness impregnation | 80, 24 | 600, 6 | 400, 2 |
| Catalyst I | Ru + Co | Zeolite 1 | 0.5 + 10 | Excessive Solution Impregnation | 120, 12 | 550, 6 | 350, 1 |
| Catalyst J | Catalyst A was obtained by reaction-regeneration treatment of catalyst A (i.e., the catalyst after the reaction in Example 1 was calcined at 550°C in air atmosphere for 2h and then reduced at 300°C in hydrogen atmosphere for 2h) | | | | | | |
| Catalyst K | Catalyst J was obtained by reaction-regeneration treatment of catalyst J (i.e., the catalyst after the reaction in Example 1 was calcined at 550°C in air atmosphere for 2h and then reduced at 300°C in hydrogen atmosphere for 2h) | | | | | | |

III. Examples of Catalytic Reactions

The catalyst of the present disclosure may be used in tank reactors.

Reaction units were equipped with a gas mass flowmeter to control gas flow, and a gas chromatograph was used for quantitative analysis of products.

A tank reactor is taken as an example:

2g of the prepared catalyst and 40g polyolefin were mixed and placed in a tank reactor, and the air in the reactor was purged with CO (0.2-6 MPa, and containing 5% Ar as an internal standard for gas chromatographic analysis). Then the temperature in the reactor was raised to 180-300 °C for reaction. The products were quantitatively analyzed by gas chromatography.

Gaseous products were analyzed with Ar as an internal standard and yields were calculated. Liquid products were collected and analyzed with n-undecane as an internal standard, and yields were calculated.

Reaction performances can be modulated by changing the reaction temperature, reaction pressure, and the feed mass ratio of CO to polyolefin.

Reaction performances are as follows: A total selectivity for aromatics can reach 60-80%, with benzene, toluene and xylene (BTX) accounting for more than 60% of aromatics. The introduction of CO not only serves as a feedstock for aromatic production but also reacts with hydrogen species generated during the aromatization of hydrogen transfer, suppressing the alkane formation and enhancing the selectivity of aromatic components.

Specific applications and effect data of catalysts are shown in Table 3.

Table 3 Specific applications and effect data of catalysts

| Example | Catalyst | Temperature (°C) | Type of Polyolefin | Mass ratio of polyolefin to catalyst | $CO_2$ Partial Pressure (MPa) | Reaction time (h) | $CO_2$ conversion rate $(g \cdot g^{-1}_{(polyolefin)} \cdot h^{-1})$ | Yield of Aromatics (%) | Proportion of BTX in aromatics (%) |
|---|---|---|---|---|---|---|---|---|---|
| 1 | Catalysts A | 280 | Polyethylene | 20 | 1 | 4 | 0.38 | 68 | 66 |
| 2 | Catalysts B | 230 | Polypropylene | 6 | 3 | 15 | 0.11 | 52 | 72 |
| 3 | Catalysts C | 300 | Waste PE cling film | 100 | 2 | 2 | 0.50 | 71 | 74 |
| 4 | Catalysts D | 270 | Polybutene | 30 | 0.5 | 5 | 0.34 | 63 | 76 |
| 5 | Catalysts E | 250 | PP plastic bottle | 15 | 5 | 5 | 0.25 | 62 | 67 |
| 6 | Catalysts F | 300 | Polyethylene | 40 | 6 | 3 | 0.56 | 75 | 65 |
| 7 | Catalysts G | 260 | PE plastic bag | 20 | 4 | 5 | 0.27 | 69 | 66 |
| 8 | Catalysts H | 200 | Polybutene | 5 | 0.5 | 20 | 0.07 | 52 | 78 |
| 9 | Catalysts I | 220 | Polypropylene | 4 | 0.8 | 15 | 0.11 | 55 | 73 |
| 10 | Catalysts J | 280 | Waste polyethylene box | 20 | 1 | 4 | 0.36 | 61 | 71 |
| 11 | Catalysts K | 280 | Polyethylene | 20 | 1 | 4 | 0.38 | 68 | 70 |
| 12 | Catalysts B | 300 | Polyethylene | 6 | 3 | 15 | 0.58 | 79 | 78 |
| 13 | Catalysts B | 280 | Polyethylene | 6 | 3 | 15 | 0.47 | 75 | 76 |
| 14 | Catalysts B | 280 | Polyethylene | 3 | 3 | 15 | 0.54 | 76 | 72 |
| 15 | Catalysts B | 280 | Polyethylene | 6 | 1 | 15 | 0.49 | 73 | 82 |
| 16 | Catalysts B | 280 | Polyethylene | 6 | 3 | 5 | 0.38 | 64 | 73 |
| Comparative | Catalysts L | 280 | Polyethylene | 20 | 1 | 4 | 0.04 | 4 | 96 |

| Example 1 | | | | | | | | | |
|---|---|---|---|---|---|---|---|---|---|
| Comparative Example 2 | Catalysts M | 280 | Polyethylene | 20 | 1 | 4 | 0.26 | 38 | 30 |
| Comparative Example 3 | Catalysts N | 280 | Polyethylene | 20 | 1 | 4 | 0.08 | 21 | 54 |
| Comparative Example 4 | Catalysts O | 280 | Polyethylene | 20 | 1 | 4 | 0.03 | 11 | 82 |
| Comparative Example 5 | Catalysts P | 280 | Polyethylene | 20 | 1 | 4 | 0.05 | 44 | 75 |
| Comparative Example 6 | Catalysts Q | 280 | Polyethylene | 20 | 1 | 4 | -- | 8 | 3 |
| Comparative Example 7 | Catalysts R | 280 | Polyethylene | 20 | 1 | 4 | 0.04 | 35 | 61 |
| Comparative Example 8 | Catalysts A | 280 | Polyethylene | $\infty$ | 30 | | -- | 0 | 0 |
| Comparative Example 9 | Catalysts A | 280 | -- | 0 | -- | | -- | 32 | 59 |
| Comparative Example 10 | Catalysts A | 280 | Polyethylene | 20 | 0.1 | 4 | 0.10 | 49 | 61 |

The metal component and preparation method of catalyst L used in comparative example 1 were the same as those of catalyst A, but the zeolite component was replaced with a commercially available zeolite 5 from Nankai University Catalyst Factory, which has a three-dimensional eight-membered ring pore structure.

The metal component and preparation method of catalyst M used in comparative example 2 were the same as those of catalyst A, but the zeolite component was replaced with a commercially available zeolite 6 from Nankai University Catalyst Factory, which has a three-dimensional twelve-membered ring pore structure.

The metal component and preparation method of catalyst N used in comparative example 3 were the same as those of catalyst A, but the zeolite component was replaced with a commercially available zeolite 7 from Nankai University Catalyst Factory, which has a one-dimensional pore structure with coexisting eight- and twelve-membered rings.

The metal component and preparation method of catalyst O used in comparative example 4 were the same as those of catalyst A, but the zeolite component was replaced with a commercially available zeolite 8 from Nankai University Catalyst Factory, which has a one-dimensional ten-membered ring pore structure.

The reaction results of Comparative Examples 1 to 4 indicate that zeolites with different topological structures have a significant effect on the selectivity of the products. The SAPO-34 zeolite having a three-dimensional eight-membered ring structure is unfavorable for polyolefin conversion and aromatic production, instead favoring generation of short-chain hydrocarbons, with an aromatic yield of only 4%. The USY zeolite having a three-dimensional twelve-membered ring structure with large pore openings, leads to the formation of heavier aromatics (e.g., naphthalene) and rapid coking deactivation, with heavy aromatics accounting for 70% of the total aromatic products. The MOR zeolite having a one-dimensional pore structure with coexisting eight- and twelve-membered rings and the SAPO-11 zeolite having a one-dimensional ten-membered ring pore structure are unfavorable for aromatization, primarily yielding cracked gasoline products with low aromatic content of 21% aromatics.

The metal component and preparation method catalyst P used in comparative example 5 were the same as those of catalyst A, but the zeolite component was replaced with zeolite 9, which has a moderate-strength acid density of only 0.07 mmol/g. The reaction performance of comparative example 5 was extremely poor, likely due to insufficient acid density for effective aromatization, highlighting the importance of adequate moderate-strength acid density.

The catalyst Q used in comparative example 6 was prepared by directly impregnating, Pt on $\gamma$-Al$_2$O$_3$. The results show that almost no CO conversion, low polyolefin conversion, and poor yields of aromatics and BTX yields.

The zeolite component of catalyst R used in comparative example 7 was the same as that of catalyst A (zeolite 1), but lacked the Pt metal component. Since the zeolite cannot activate CO, only olefins underwent aromatization, with a low aromatic yield of 38%. Comparative example 7 primarily produced long-chain alkanes, with a low CO conversion rate of only 0.02 g·g$^{-1}$ $_{(polyolefin)}$·h$^{-1}$, which fails to achieve high selectivity for aromatics or effective utilization of CO.

Comparative examples 6 and 7 show that when only one functional component, either metal or zeolite, is present, the reaction performance is poor and far inferior to the excellent reaction performance described in the present disclosure.

Comparative example 8 and example 1 both employed the catalyst A, but no polyolefin was added in the comparative example 8. The results show that CO cannot be effectively converted without polyolefin.

Comparative example 9 and example 1 both employed the catalyst A, but no CO was added in comparative example 9 and N$_2$ was used as the pressurizing gas. The results show that polyolefin can still be converted to aromatics by the bifunctional catalyst without CO, but the aromatic yield was low (36%), similar to comparative example 7, and far inferior to the excellent reaction performance described in the present disclosure.

It can be seen from the reaction results of comparative examples 8 and 9 that the conversion of CO and polyolefin are mutually coupled and synergistic. Without polyolefin, CO cannot be effectively converted; and without CO, polyolefin cannot be highly selectively converted into aromatics, and the amount of alkane byproducts increases.

Comparative example 10 and example 1 both employed the catalyst A, but the CO partial pressure was lower in comparative example 10. Insufficient CO weakened its promoting effect on aromatic selectivity, emphasizing the importance of adequate CO supply for optimal catalytic performance.

The above results indicate that the topological structure and acid properties of the zeolites, the feed ratio of CO to polyolefin, and the synergy between the metal/metal oxide and the zeolites play critical roles in determining aromatic selectivity, CO conversion rate, and the content of benzene, toluene, and xylene in the aromatic products.

**Claims**

1. A method for preparing aromatic hydrocarbons by means of coupling conversion of carbon monoxide and polyolefin, comprising:

   using carbon dioxide and polyolefin plastics as reaction raw materials and employing a bifunctional catalyst, wherein one functional component is selected from a metal, a metal oxide, and a mixture thereof, and another functional component comprises a zeolite;
   wherein,
   the zeolite has an MFI, MEL, or MWW topological structure;
   the metal/metal oxide comprise one or more elements selected from Fe, Co, Ni, Mn, Ce, Cu, Ru, Zn, Ga, Rh, Pd, Ir, and Pt.

2. The method according to claim 1, wherein a pressure of the carbon dioxide is 0.2-6 MPa, a reaction temperature is 180-400°C, and a mass ratio of catalyst to polyolefin is ≥1:500.

3. The method according to claim 1, wherein a mass fraction of the metal or metal oxide in the bifunctional catalyst is 0.1 wt% - 60 wt%, wherein the metal or metal oxide is either directly loaded onto the zeolite, or is dispersed on a metal oxide carrier and then physically mixed with the zeolite to form the bifunctional catalyst, wherein the metal oxide carrier is selected from Al$_2$O$_3$, TiO$_2$, ZrO$_2$, CeO$_2$, ZnO, MoO$_x$, MnO$_x$, and mixtures thereof.

4. The method according to claim 1, wherein the zeolite having an MFI, MEL, or MWW topological structure has a framework comprising at least one of Si-O, Si-O-Al, Si-O-Al-P, Si-O-Al-B, and Si-O-Al-Ge, and the zeolite having an MFI, MEL, or MWW topological structure is preferably at least one of MCM-22, ZSM-11 and ZSM-5.

5. The method according to claim 1, wherein the zeolite having an MFI, MEL, or MWW topological structure contains moderate-strength acid sites in an amount of ≥0.1 mol/kg.

6. The method according to claim 1, wherein the metal or metal oxide and the zeolite are compounded in a mechanical mixing or loading mode, wherein the loading mode is to load the metal or metal oxide onto the zeolite via impregnation, deposition precipitation, or vapor deposition.

7. The method according to claim 1, wherein the metal/metal oxide component in the catalyst activates CO, and the zeolite activates polyolefin.

8. The method according to claim 1, wherein the bifunctional catalyst is pre-reduced in H$_2$ or CO atmosphere at 300-500 °C for 0.5-10 h.

**9.** The method according to claim 2, wherein,

when the reaction is carried out in a tank reactor, a residence time of reactants is 0.5-20 h;
when the reaction is carried out in a moving-bed reactor, a gas hourly space velocity is 20-2000 mL·g$^{-1}$·h$^{-1}$.

**10.** The method according to claim 9, wherein the polyolefin comprises at least one of polyethylene, polypropylene, and polybutene, as well as plastic products made therefrom or post-consumer waste plastics.

## INTERNATIONAL SEARCH REPORT

| International application No. |
|---|
| **PCT/CN2023/128160** |

| A. | CLASSIFICATION OF SUBJECT MATTER |
|---|---|

B01J29/44(2006.01)i; C10G1/10(2006.01)i; C10G2/00(2006.01)i; B01J29/76(2006.01)i; B01J29/48(2006.01)i; B01J29/40(2006.01)i; B01J29/46(2006.01)i; C07C2/86(2006.01)i; C07C15/06(2006.01)i; C07C15/04(2006.01)i; C07C15/08(2006.01)i

According to International Patent Classification (IPC) or to both national classification and IPC

| B. | FIELDS SEARCHED |
|---|---|

Minimum documentation searched (classification system followed by classification symbols)

IPC: B01J; C10G; C07C

Documentation searched other than minimum documentation to the extent that such documents are included in the fields searched

Electronic data base consulted during the international search (name of data base and, where practicable, search terms used)

CNTXT, ENTXT, DWPI, CNKI, Caplus (STN): 中国科学院大连化学物理研究, 潘秀莲, 丁一, 邵宇, 包信和, 一氧化碳, 聚烯烃, 苯, 甲苯, 二甲苯, 芳烃, 耦合, 催化, 分子筛, CO, carbon monoxide, polyolefin, benzene, toluene, xylene, BTX, aromatic?, coupl?, cataly?, molecular sieves

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 115161051 A (GUANGZHOU INSTITUTE OF ENERGY CONVERSION, CHINESE ACADEMY OF SCIENCES) 11 October 2022 (2022-10-11) abstract, and description, paragraphs [0007]-[0021] | 1-10 |
| Y | JP 2001334151 A (ICHIKAWA, M. et al.) 04 December 2001 (2001-12-04) abstract | 1-10 |
| A | CN 103484142 A (TSINGHUA UNIVERSITY) 01 January 2014 (2014-01-01) entire document | 1-10 |
| A | CN 110819372 A (GUANGZHOU INSTITUTE OF ENERGY CONVERSION, CHINESE ACADEMY OF SCIENCES) 21 February 2020 (2020-02-21) entire document | 1-10 |
| A | CN 116173935 A (DALIAN INSTITUTE OF CHEMICAL PHYSICS, CHINESE ACADEMY OF SCIENCES) 30 May 2023 (2023-05-30) entire document | 1-10 |

☑ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **22 May 2024** | **28 May 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/128160**

| C. | DOCUMENTS CONSIDERED TO BE RELEVANT | |
|---|---|---|
| Category* | Citation of document, with indication, where appropriate, of the relevant passages | Relevant to claim No. |
| A | US 2022195310 A1 (BIOBTX B. V.) 23 June 2022 (2022-06-23)<br>entire document | 1-10 |

Form PCT/ISA/210 (second sheet) (July 2022)

## INTERNATIONAL SEARCH REPORT
### Information on patent family members

International application No.

**PCT/CN2023/128160**

| Patent document cited in search report | | | Publication date (day/month/year) | Patent family member(s) | | | Publication date (day/month/year) |
|---|---|---|---|---|---|---|---|
| CN | 115161051 | A | 11 October 2022 | None | | | |
| JP | 2001334151 | A | 04 December 2001 | None | | | |
| CN | 103484142 | A | 01 January 2014 | None | | | |
| CN | 110819372 | A | 21 February 2020 | None | | | |
| CN | 116173935 | A | 30 May 2023 | None | | | |
| US | 2022195310 | A1 | 23 June 2022 | ZA | 202107521 | B | 28 June 2023 |
| | | | | IL | 286790 | A | 31 October 2021 |
| | | | | JP | 2022528272 | A | 09 June 2022 |
| | | | | EP | 3947603 | A1 | 09 February 2022 |
| | | | | WO | 2020204707 | A1 | 08 October 2020 |

Form PCT/ISA/210 (patent family annex) (July 2022)

**REFERENCES CITED IN THE DESCRIPTION**

**Non-patent literature cited in the description**

- **FAN ZHANG et al.** *Science*, 2020, vol. 370, 437-441